# EUROPÄISCHE PATENTANMELDUNG

(11) **EP 4 011 892 A1**
(43) Veröffentlichungstag der Anmeldung: **15.06.2022**
(21) Anmeldenummer: 20212752.8
(22) Anmeldetag: 09.12.2020
(51) Int. Cl.: C07F 15/00, C07C 51/00, C07C 53/126

(54) **PLATIN-KOMPLEXE MIT FERROCEN-DIPHOSPHIN-LIGANDEN FÜR DIE KATALYSE DER HYDROXYCARBONYLIERUNG ETHYLENISCH UNGESÄTTIGTER VERBINDUNGEN**

(71) Anmelder: Evonik Operations GmbH, 45128 Essen (DE)
(72) Erfinder: SCHNEIDER, Carolin, 40789 Monheim an Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(57) **Zusammenfassung**

Platin-Komplexe mit Ferrocen-Diphosphin-Liganden für die Katalyse der Hydroxycarbonylierung ethylenisch ungesättigter Verbindungen.

## Beschreibung

Die vorliegende Erfindung betrifft Platin-Komplexe mit Ferrocen-Diphosphin-Liganden für die Katalyse der Hydroxycarbonylierung ethylenisch ungesättigter Verbindungen.

Die Hydroxycarbonylierung ethylenisch ungesättigter Verbindungen ist ein Prozess mit steigender Bedeutung. Unter einer Hydroxycarbonylierung versteht man die direkte Umsetzung von ethylenisch ungesättigten Verbindungen, wie Olefinen, mit Kohlenmonoxid in Gegenwart eines Metalls bzw. eines Metallkomplexes und eines Liganden zu den entsprechenden Säuren:

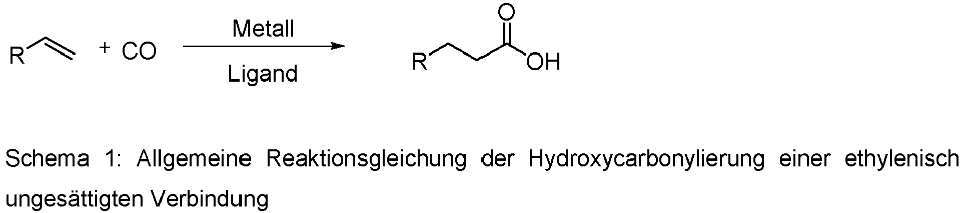

In EP 3121186 A2 werden ferrocenbasierte Verbindungen und deren Verwendung in der Alkoxycarbonylierung beschrieben. Als Katalysator wird hierbei ein Pd-Komplex eingesetzt, welcher diese Liganden umfasst.

Ein Nachteil von Palladium ist dessen hoher Preis.

Die technische Aufgabe, welche der vorliegenden Erfindung zugrunde lag, ist die Bereitstellung neuer Komplexe, welche ein Metall als Zentralatom aufweisen, welches einen niedrigeren Preis als Palladium hat. Mit den Komplexen sollen zudem bei Hydroxycarbonylierungen gute Umsätze erzielt werden.

Diese Aufgabe wird gelöst durch einen Komplex nach Anspruch 1.

Komplex umfassend Pt und eine Verbindung gemäß Formel (**I**) 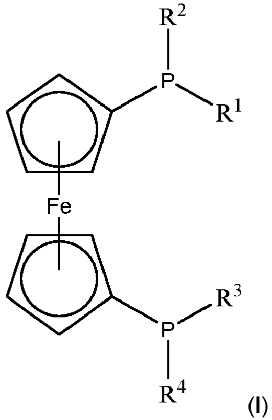 wobei
R¹, R², R³, R⁴ jeweils unabhängig voneinander ausgewählt sind aus -(C₁-C₁₂)-Alkyl, -(C₆-C₂₀)-Heteroaryl.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₆-C₂₀)-Heteroaryl umfasst mono- oder polycyclische aromatische Kohlenwasserstoffreste mit 6 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 6 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest.

Geeignete (C₆-C₂₀)-Heteroarylgruppen mit mindestens sechs Ringatomen sind insbesondere Pyridyl, Pyridazinyl, Pyrimidyl, Pyrazinyl, Benzofuranyl, Indolyl, Isoindolyl.

In einer Ausführungsform stehen mindestens zwei der Reste R¹, R², R³, R⁴ für einen -(C₆-C₂₀)-Heteroarylrest mit mindestens sechs Ringatomen.

In einer Ausführungsform stehen die Reste R¹ und R³ jeweils für einen -(C₆-C₂₀)-Heteroarylrest mit mindestens sechs Ringatomen.
In einer Ausführungsform stehen die Reste R¹ und R³ jeweils für 2-Pyridyl.

In einer Ausführungsform stehen R² und R⁴ für -(C₁-C₁₂)-Alkyl.

In einer Ausführungsform stehen R² und R⁴ für *tert*-Butyl.

In einer Ausführungsform weist die Verbindung (**I**) die Struktur (**1**) auf:

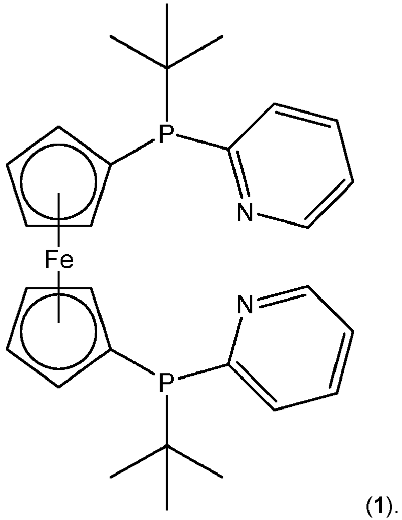

Die Erfindung betrifft weiterhin die Verwendung eines erfindungsgemäßen Komplexes zur Katalyse einer Hydroxycarbonylierungsreaktion.

Verfahren umfassend die Verfahrensschritte:
a) Vorlegen einer ethylenisch ungesättigten Verbindung;
b) Zugabe eines wie zuvor beschriebenen Komplexes, oder eine Verbindung gemäß Formel (**I**) 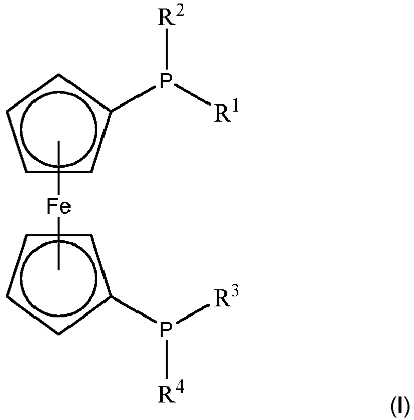 wobei
   R¹, R², R³, R⁴ jeweils unabhängig voneinander ausgewählt sind aus -(C₁-C₁₂)-Alkyl, -(C₆-C₂₀)-Heteroaryl und
   einer Substanz, welche Pt umfasst;
c) Zugabe einer Säure;
d) Zuführen von CO;
e) Erwärmen des Reaktionsgemisches aus a) bis d), wobei die ethylenisch ungesättigte Verbindung zu einer Carbonsäure umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

In einer Variante des Verfahrens umfasst die ethylenisch ungesättigte Verbindung keine weiteren funktionellen Gruppen außer Kohlenstoff-Kohlenstoff-Doppelbindungen.

In einer Variante des Verfahrens ist die ethylenisch ungesättigte Verbindung ausgewählt aus: Ethen, Propen, 1-Buten, *cis-* und/oder *trans*-2-Buten, iso-Buten, 1,3-Butadien, 1-Penten, *cis-*und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

In einer Variante des Verfahrens ist die Säure in Verfahrensschritt c) ausgewählt aus: Essigsäure, Perchlorsäure, Schwefelsäure, Phosphorsäure, Methylphosphonsäure, Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure, Dodecylsulfonsäure, Camphersulfonsäure.

In einer Variante des Verfahrens ist die Säure in Verfahrensschritt c) Essigsäure (AcOH).

In einer Variante des Verfahrens ist die Substanz, welche Pt umfasst ausgewählt aus: Platindichlorid (PtCl₂), Platin(II)-acetylacetonat [Pt(acac)₂], Platin(II)-acetat [Pt(OAc)₂], Dichloro(1,5-cyclooctadiene)platin(II) [Pt(cod)₂Cl₂], Bis(dibenzylideneaceton)platin [Pt(dba)₂], Bis(acetonitrile)dichloropatin(II) [Pt(CH₃CN)₂Cl₂], Platin(cinnamyl)dichlorid [Pt(cinnamyl)Cl₂].

In einer Variante des Verfahrens ist die Substanz, welche Pt umfasst ausgewählt aus: Platindichlorid (PtCl₂), Platin(II)-acetylacetonat [Pt(acac)₂], Platin(II)-acetat [Pt(OAc)₂].

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 6 MPa (20 bis 60 bar) zugeführt.

Die Reaktionsmischung wird in Schritt e) des erfindungsgemäßen Verfahrens vorzugsweise auf eine Temperatur im Bereich von 60 °C bis 160 °C, bevorzugt von 80 °C bis 140 °C, besonders bevorzugt von 80 °C bis 120 °C erwärmt, um die ethylenisch ungesättigte Verbindung zu einer Säure umzusetzen.

Im Folgenden soll die Erfindung anhand eines Ausführungsbeispieles näher erläutert werden.

### Umsetzung von 1-Octen zur Säure

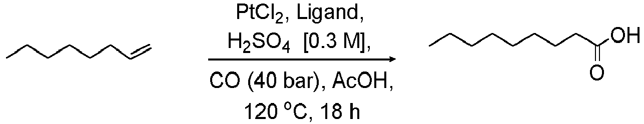

Reaktionsbedingungen: 1-Octen (1.0 mmol), PtCl₂ (0.01 mmol, 1.0 mol%), Ligand: bidentater Phosphinligand (0.022 mmol, 2.2 mol%), Schwefelsäure [0.3 M] 0.5 mL, AcOH (1.5 mL), Druck (CO): 40 bar, Temperatur: 120 °C, Reaktionszeit: 18 h.

Die Reaktion wurde in einem erfindungsgemäßen Verfahren mit dem Ligand (1), sowie als Vergleichsversuch mit dem Liganden (2) durchgeführt:

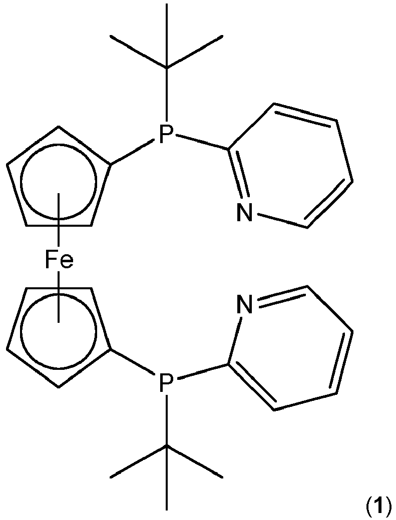

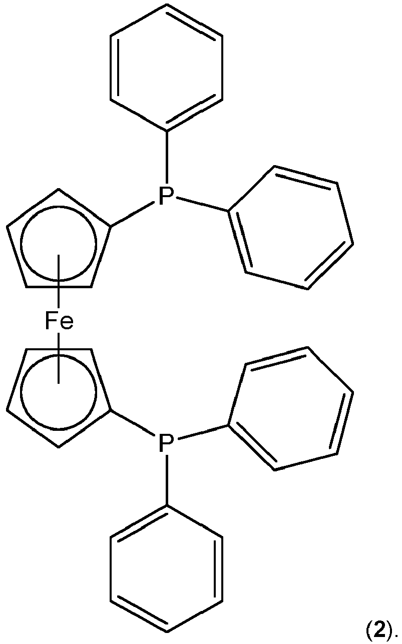

Das erfindungsgemäße Verfahren mit Ligand (**1**) lieferte hierbei eine Ausbeute von 41%. In dem Vergleichsversuch mit Ligand (**2**) konnte hingegen nur eine Ausbeute von 13% erzielt werden.

Der Preis von Pt liegt unter dem von Pd. Die Aufgabe wird somit durch einen erfindungsgemäßen Komplex gelöst.

## Patentansprüche

1. Komplex umfassend Pt und eine Verbindung gemäß Formel (**I**) 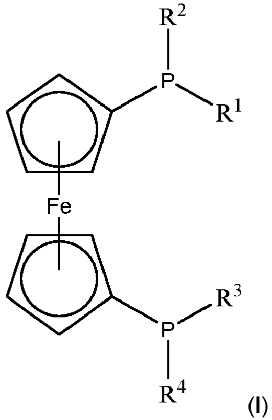 wobei
R¹, R², R³, R⁴ jeweils unabhängig voneinander ausgewählt sind aus -(C₁-C₁₂)-Alkyl, - (C₆-C₂₀)-Heteroaryl.

2. Komplex nach Anspruch 1,
wobei mindestens zwei der Reste R¹, R², R³, R⁴ für einen -(C₆-C₂₀)-Heteroarylrest mit mindestens sechs Ringatomen stehen.

3. Komplex nach einem der Ansprüche 1 bis 2,
wobei die Reste R¹ und R³ jeweils für einen -(C₆-C₂₀)-Heteroarylrest mit mindestens sechs Ringatomen stehen.

4. Komplex nach einem der Ansprüche 1 bis 3,
wobei die Reste R¹ und R³ jeweils für 2-Pyridyl stehen.

5. Komplex nach einem der Ansprüche 1 bis 4,
wobei R² und R⁴ für -(C₁-C₁₂)-Alkyl stehen.

6. Komplex nach einem der Ansprüche 1 bis 5,
wobei R² und R⁴ für *tert*-Butyl stehen.

7. Komplex nach einem der Ansprüche 1 bis 6,
wobei die Verbindung (**I**) die Struktur (1) aufweist:

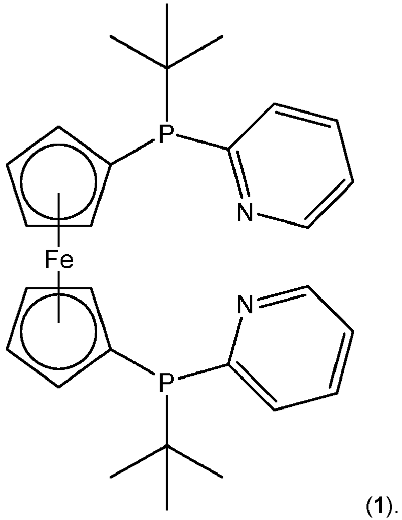

8. Verfahren umfassend die Verfahrensschritte:
a) Vorlegen einer ethylenisch ungesättigten Verbindung;
b) Zugabe eines Komplexes nach einem der Ansprüche 1 bis 7, oder eine Verbindung gemäß Formel (**I**) 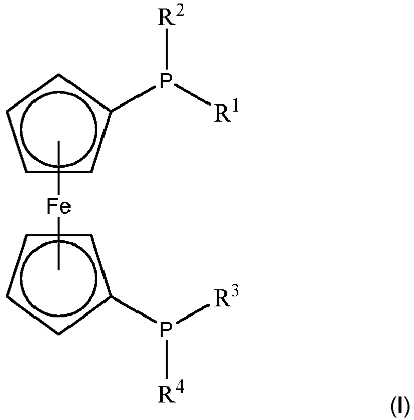 wobei
R¹, R², R³, R⁴ jeweils unabhängig voneinander ausgewählt sind aus -(C₁-C₁₂)-Alkyl, - (C₆-C₂₀)-Heteroaryl und
einer Substanz, welche Pt umfasst;
c) Zugabe einer Säure;
d) Zuführen von CO;
e) Erwärmen des Reaktionsgemisches aus a) bis d), wobei die ethylenisch ungesättigte Verbindung zu einer Carbonsäure umgesetzt wird.

9. Verfahren nach Anspruch 8,
wobei die ethylenisch ungesättigte Verbindung ausgewählt ist aus: Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

10. Verfahren nach einem der Ansprüche 8 oder 9,
wobei die Säuere in Verfahrensschritt c) ausgewählt ist aus: Essigsäure, Perchlorsäure, Schwefelsäure, Phosphorsäure, Methylphosphonsäure, Methansulfonsäure, Trifluormethansulfonsäure, tert-Butansulfonsäure, p-Toluolsulfonsäure, 2-Hydroxypropan-2-sulfonsäure, 2,4,6-Trimethylbenzolsulfonsäure, Dodecylsulfonsäure, Camphersulfonsäure.

11. Verfahren nach einem der Ansprüche 8 bis 10,
wobei die Säure in Verfahrensschritt c) Essigsäure ist.

12. Verfahren nach einem der Ansprüche 8 bis 11,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂], Dichloro(1,5-cyclooctadiene)platin(II) [Pt(cod)₂Cl₂], Bis(dibenzylideneaceton)platin [Pt(dba)₂], Bis(acetonitrile)dichloropatin(II) [Pt(CH₃CN)₂Cl₂], Platin(cinnamyl)dichlorid [Pt(cinnamyl)Cl₂].

13. Verfahren nach einem der Ansprüche 8 bis 12,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂].

## Geänderte Patentansprüche

### Geänderte Patentansprüche gemäss Regel 137(2) EPÜ.

1. Verfahren umfassend die Verfahrensschritte:
a) Vorlegen einer ethylenisch ungesättigten Verbindung;
b) Zugabe einer Verbindung gemäß Formel (**I**) 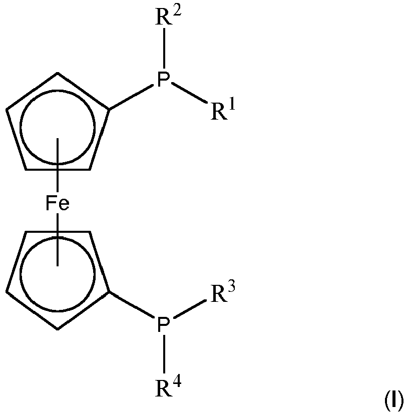 wobei
R¹, R², R³, R⁴ jeweils unabhängig voneinander ausgewählt sind aus -(C₁-C₁₂)-Alkyl,-(C₆-C₂₀)-Heteroaryl und
einer Substanz, welche Pt umfasst;
c) Zugabe von Essigsäure;
d) Zuführen von CO;
e) Erwärmen des Reaktionsgemisches aus a) bis d), wobei die ethylenisch ungesättigte Verbindung zu einer Carbonsäure umgesetzt wird.

2. Verfahren nach Anspruch 1,
wobei die ethylenisch ungesättigte Verbindung ausgewählt ist aus: Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

3. Verfahren nach einem der Ansprüche 2 oder 3,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂], Dichloro(1,5-cyclooctadiene)platin(II) [Pt(cod)₂Cl₂], Bis(dibenzylideneaceton)platin [Pt(dba)₂], Bis(acetonitrile)dichloropatin(II) [Pt(CH₃CN)₂Cl₂], Platin(cinnamyl)dichlorid [Pt(cinnamyl)Cl₂].

4. Verfahren nach einem der Ansprüche 1 bis 3,
wobei die Substanz, welche Pt umfasst ausgewählt ist aus: Platindichlorid (PtCl₂), Platin(II)-acetylacetonat [Pt(acac)₂], Platin(II)-acetat [Pt(OAc)₂].
